(19) Europäisches Patentamt
European Patent Office
Office européen des brevets

(11) Veröffentlichungsnummer: 0 226 197
A2

# (12) EUROPÄISCHE PATENTANMELDUNG

(21) Anmeldenummer: 86117351.6

(51) Int. Cl.4: **A61K 7/13** , C09B 55/00

(22) Anmeldetag: 12.12.86

(30) Priorität: 20.12.85 DE 3545245

(43) Veröffentlichungstag der Anmeldung: 24.06.87 Patentblatt 87/26

(84) Benannte Vertragsstaaten: AT BE CH DE FR GB IT LI NL SE

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Konrad, Günther, Dr.**
**Feuerbachweg 12**
**D-4010 Hilden(DE)**
Erfinder: **Möller, Hinrich, Dr.**
**Schumannstrasse 11**
**D-4019 Monheim(DE)**
Erfinder: **Lieske, Edgar**
**Hunsrückenstrasse 40**
**D-4000 Düsseldorf(DE)**

## (54) Haarfärbemittel mit direktziehenden Azomethinfarbstoffen.

(57) Haarfärbemittel enthalten als direktziehende Haarfarbstoffe Azomethinfarbstoffe der Formel

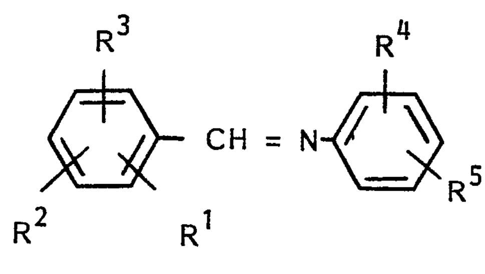

in der wenigstens zwei der Gruppen $R^1$ bis $R^3$ Hydroxylgruppen sind und die dritte gegebenenfalls Wasserstoff oder eine Alkylgruppe mit 1 bis 4 C-Atomen ist, $R^4$ Wasserstoff oder eine Alkylgruppe mit 1 bis 4 C-Atomen und $R^5$ eine Gruppe $-OR^6$ oder $-NR^7R^8$, worin $R^6$, $R^7$ und $R^8$ Wasserstoff, Alkylgruppen mit 1 bis 4 C-Atomen oder Hydroxyalkylgruppen mit 2 bis 4 C-Atomen sind. Diese Farbstoffe besitzen ein gutes Aufziehvermögen auf Keratinfasern und erzeugen gelbe Nuancen von guter Intensität und Echtheit.

EP 0 226 197 A2

Xerox Copy Centre

## "Haarfärbemittel mit direktziehenden Azomethinfarbstoffen"

Gegenstand der Erfindung sind Haarfärbemittel mit einem Gehalt an direktziehenden Haarfarbstoffen. Solche Haarfärbemittel enthalten direktziehende Haarfarbstoffe in einem kosmetischen Träger. Oft enthalten solche Haarfärbemittel zur Erzielung bestimmter Nuancen zusätzlich Oxidationsfarbstoffvorprodukte. Als kosmetische Träger für die direktziehenden Haarfarbstoffe und gegebenenfalls Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haar spielen neben den Oxidationsfarben, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen, Triphenylmethanfarbstoffen, kationischen Azofarbstoffen oder mit Oxidationsfarbstoffen eingesetzt.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen - schließlich in toxikologischer und dermatologischer Hinsicht unbedenklich sein.

Leider sind zahlreiche Farbstoffe vom Typ der Nitrobenzolderivate toxikologisch und dermatologisch bedenklich. Es besteht daher ein dringendes Bedürfnis an direktziehenden Haarfarbstoffen, die nicht zur Gruppe der Nitroaniline gehören und deren Verwendung als Haarfärbemittel nicht mit toxikologischen Risiken verbunden ist.

Die bekannten Azomethinfarbstoffe haben sich als nicht genügend farbintensiv oder auf Keratinfasern als zu schwach aufziehend erwiesen, und ihre Verwendung zum Färben von menschlichen Haaren ist daher bisher nicht vorgeschlagen worden. Es wurde nunmehr eine Gruppe von Azomethinfarbstoffen gefunden, die auf Keratinfasern ein gutes Aufziehvermögen besitzen und die dem damit gefärbten Haar intensive, wasch-und lichtechte gelbe Nuancen verleihen und die sich daher in besonderer Weise für die Verwendung zum Färben menschlicher Haare eignen.

Gegenstand der Erfindung sind daher Haarfärbemittel mit einem Gehalt an direktziehenden Haarfarbstoffen, die als direktziehende Haarfarbstoffe eine oder mehrere Verbindungen der Formel I enthalten,

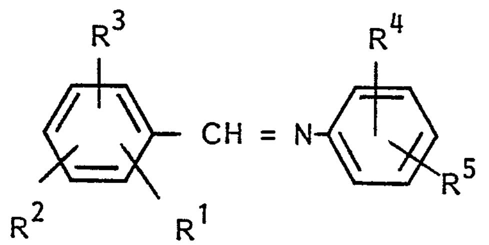

(I)

in der wenigstens zwei der Gruppen $R^1$ bis $R^3$ Hydroxylgruppen sind und die dritte gegebenenfalls Wasserstoff oder eine Alkylgruppe mit 1 bis 4 C-Atomen ist, $R^4$ Wasserstoff oder eine Alkylgruppe mit 1 bis 4 C-Atomen und $R^5$ eine Gruppe $-OR^6$ oder eine Gruppe $-NR^7R^8$ ist, worin $R^6$, $R^7$ und $R^8$ Wasserstoff, Alkylgruppen mit 1 bis 4 C-Atomen oder Hydroxyalkylgruppen mit 2 bis 4 C-Atomen sind.

Die Gruppen $R^1$ bis $R^3$ können in beliebiger Position zueinander und zur Azomethingruppe angeordnet sein. Bevorzugt werden jedoch solche Verbindungen der Formel I verwendet, bei welchen wenigstens eine der Hydroxylgruppen in Ortho-oder Para-Position zur Azomethingruppe steht. Besonders günstig verhalten sich solche Verbindungen der Formel I, bei welchen wenigstens eine Hydroxylgruppe in Ortho-Position zur Azomethingruppe steht.

Wegen ihrer leichteren technischen Zugänglichkeit sind weiterhin solche Azomethine der Formel I bevorzugt, in welchen wenigstens zwei der Gruppen $R^1$ bis $R^3$ Hydroxylgruppen und die dritte Wasserstoff ist, $R^4$ Wasserstoff ist, $R^5$ in Para-Position zur Azomethingruppe steht und eine Gruppe $-OR^6$ oder $-NR^7R^8$ ist und $R^6$, $R^7$ und $R^8$ Alkylgruppen oder Hydroxyalkylgruppen sind.

Die Verbindungen der Formel I lassen sich, soweit sie nicht literaturbekannt sind, nach literaturbekannten Verfahren herstellen. Das Verfahren zur Herstellung besteht ganz allgemein in einer Kondensation des entsprechenden Hydroxybenzaldehyds der Formel II

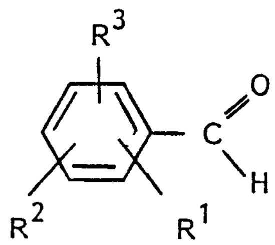

(II)

mit einem Anilinderivat der Formel III

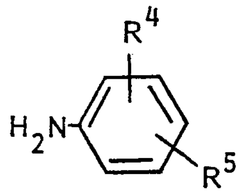

(III)

wobei in den Formeln II und III die Gruppen $R^1$ bis $R^5$ die für Formel I angegebene Bedeutung haben.

Die Verbindungen der Formel I sind in toxikologischer und dermatologischer Hinsicht unschädlich und daher für die Anwendung in Haarfärbemitteln besonders geeignet.

Die erfindungsgemäßen Haarfärbemittel können die direktziehenden Azomethinfarbstoffe der allgemeinen Formel I allein oder in Kombination mit bekannten direktziehenden Farbstoffen, z. B. mit Nitrobenzolderivaten, Anthrachinonfarbstoffen, Triphenylmethan-oder Azofarbstoffen enthalten. Darüber hinaus eignen sich die direktziehenden Farbstoffe der allgemeinen Formel I wegen ihrer hohen Beständigkeit gegenüber Reduktionsmitteln und Oxidationsmitteln auch sehr gut zur Kombination mit Oxidationsfarbstoffvorprodukten, also zur Modifikation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para-oder Orthoposition befindlichen freien oder substituierten Hydroxy-oder Aminogruppe, Diaminopyridinderivate, heterocyclische Hydrazone, 4-Aminopyrazolonderivate und 2,4,5,6-Tetraaminopyrimidin und dessen Derivate eingesetzt. Als Kupplersub stanzen werden m-Phenylendiaminderivate, Naphthole, Resorcin und Resorcinderivate, Pyrazolone und m-Aminophenole verwendet.

Zur Herstellung der erfindungsgemäßen Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz-und Emulgiermittel wie anionische, nichtionische oder ampholytische Tenside, z. B. Fettalkoholsulfate, Alkansulfonate, alpha-Olefinsulfonate, Fettalkoholpolyglykolethersulfate, Ethylenoxidanlagerungsprodukte an Fettalkohole, an Fettsäuren und an Alkylphenole, Sorbitanfettsäureester und Fettsäurepartialglyceride, Fettsäurealkanolamide sowie Verdickungsmittel wie z. B. Methyl-oder Hydroxyethylcellulose, Stärke, Fettalkohole, Paraffinöle, Fettsäuren, ferner Parfümöle und haarpflegende Zusäzte, wie z. B. wasserlösliche kationische Polymere, Proteinderivate,

Pantothensäure und Cholesterin. Die Bestandteile der kosmetischen Träger werden zur Herstellung der erfindungsgemäßen Haarfärbemittel in für diese Zwecke üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

In den erfindungsgemäßen Haarfärbemitteln werden die direktziehenden Haarfarbstoffe der allgemeinen Formel I in einer Menge von 0,01 bis 5,0 Gew.-%, bevorzugt von 0,1 bis 2 Gew.-%, bezogen auf das gesamte Haarfärbemittel, eingesetzt. Daneben können bekannte Oxidationshaarfärbemittelvorprodukte - (Entwickler-und Kupplersubstanzen) in einer Menge von 0,01 bis 5, vorzugsweise von 1 bis 3 Gew.-% enthalten sein.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den erfindungsgemäßen Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponieren erzielen. Die nachfolgenden Beispiele sollen den Erfindungsgegenstand näher erläutern, ohne ihn jedoch hierauf zu beschränken.

## Beispiele

1. Herstellung direktziehender Azomethine

Allgemeine Vorschrift:

Die Produkte 2 bis 6, 8 und 9 der Tabelle I wurden durch Vereinigung ethanolischer Lösungen molarer Mengen des entsprechenden Hydroxybenzaldehyds und des entsprechenden Anilins hergestellt. Die Azomethine fielen nach einigen Stunden bei 20 °C aus der Lösung aus und wurden abfiltriert und mit Ethanol gewaschen und getrocknet.

Die Produkte 1 und 7 der Tabelle I wurden durch Kondensation molarer Mengen des entsprechenden Hydroxybenzaldehyds und des entsprechenden Anilins in Toluol in Gegenwart von 3 Mol% p-Toluolsulfonsäure unter azeotroper Wasserabspaltung hergestellt.

2. Haarfärbeversuche

Es wurden Haarfärbecremes der folgenden Zusammensetzung hergestellt:

| | |
|---|---|
| Fettalkohol $C_{12-18}$ | 10g, |
| Fettalkohol $Cl_{12-14}$ + 2 EO-sulfat, | |
| Na-Salz (28%ig) | 25 g |
| Wasser | 60 g |
| direktziehender Farbstoff | 1 g |
| Ammoniumsulfat | 1 g |
| konzentrierte Ammoniak-Lösung | bis pH = 9,5 |
| Wasser | ad 100 g |

Die Bestandteile wurden der Reihe nach miteinander vermischt. Nach Zugabe der direktziehenden Farbstoffe wurde mit konzen trierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als direktziehende Haarfarbstoffe wurden die Verbindungen gemäß Beispiele 1 bis 9 der Tabelle I eingesetzt.

Das Ergebnis der Färbeversuche ist der Tabelle I zu entnehmen.

Tabelle I

| Nr. | Substanz | Schmelzpunkt | | Analyse % C | % H | % N | Nuance des gefärbten Haars |
|---|---|---|---|---|---|---|---|
| 1.) | 3,4-Dihydroxybenzyliden-p-anisidin | 158°C | ber.<br>gef. | 69,12<br>69,6 | 5,38<br>5,36 | 5,76<br>5,04 | bernsteingelb |
| 2.) | 2,3,4-Trihydroxybenzyliden-p-anisidin | 183°C | ber.<br>gef. | 64,86<br>64,5 | 5,02<br>5,07 | 5,44<br>5,35 | goldbraun |
| 3.) | 2,5-Dihydroxybenzyliden-p-anisidin | 147°C | ber.<br>gef. | 69,12<br>69,1 | 5,38<br>5,49 | 5,76<br>5,70 | zitronengelb |
| 4.) | 2,3-Dihydroxybenzyliden-p-anisidin | 127°C | ber.<br>gef. | 69,12<br>68,6 | 5,38<br>5,44 | 5,76<br>5,18 | buttergelb |
| 5.) | N,N-Dimethyl-N'-(2,4-dihydroxybenzyliden)-p-phenylendiamin | 210°C | ber.<br>gef. | 70,29<br>70,5 | 6,29<br>6,51 | 10,93<br>11,0 | olivgelb |
| 6.) | 2,4-Dihydroxybenzyliden-p-aminophenol | 228°C | ber.<br>gef. | 68,11<br>67,5 | 4,84<br>5,07 | 6,11<br>5,76 | zitronengelb |
| 7.) | 2,4-Dihydroxybenzyliden-p-anisidin | ab 90°C | ber.<br>gef. | 69,12<br>70,00 | 5,38<br>5,37 | 5,76<br>5,98 | olivgelb |
| 8.) | N,N-Bis-(2-hydroxyethyl)-N'-(2,4-dihydroxybenzyliden)-p-phenylendiamindihydrat | 212°C | ber.<br>gef. | 57,94<br>57,30 | 6,87<br>6,63 | 7,95<br>7,71 | olivgelb |
| 9.) | 2,4-Dihydroxybenzyliden-m-anisidin | 151°C | ber.<br>gef. | 69,12<br>69,30 | 5,38<br>5,56 | 5,76<br>5,67 | tiefgelb |

**Ansprüche**

1. Haarfärbemittel mit einem Gehalt an direktziehenden Haarfarbstoffen, dadurch gekennzeichnet, daß als direktziehende Haarfarbstoffe eine oder mehrere Verbindungen der Formel I enthalten sind,

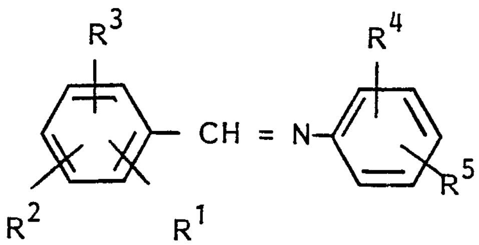

(I)

in der wenigstens zwei der Gruppen $R^1$ bis $R^3$ Hydroxylgruppen sind und die dritte gegebenenfalls Wasserstoff oder eine Alkylgruppe mit 1 bis 4 C-Atomen ist, $R^4$ Wasserstoff oder eine Alkylgruppe mit 1 bis 4 C-Atomen und $R^5$ eine Gruppe $-OR^6$ oder eine Gruppe $-NR^7R^8$ ist, worin $R^6$, $R^7$ und $R^8$ Wasserstoff, Alkylgruppen mit 1 bis 4 C-Atomen oder Hydroxyalkylgruppen mit 2 bis 4 C-Atomen sind.

2. Haarfärbemittel nach Anspruch 1, dadurch gekennzeichnet, daß wenigstens eine der Hydroxylgruppen in Ortho-oder Para-Position zur Azomethingruppe steht.

3. Haarfärbemittel nach den Ansprüchen 1 oder 2, dadurch gekennzeichnet, daß zwei der Gruppen $R^1$ bis $R^3$ Hydroxylgruppen sind und die dritte Wasserstoff ist, $R^4$ Wasserstoff ist, $R^5$ in Para-Position zur Azomethingruppe steht und eine Gruppe $-OR^6$ oder $-NR^7R^8$ ist und $R^6$, $R^7$ und $R^8$ Alkylgruppen oder Hydroxyalkylgruppen sind.

4. Haarfärbemittel nach den Ansprüchen 1 bis 3, dadurch gekennzeichnet, daß die direktziehenden Haarfarbstoffe der Formel I in einer Menge von 0,01 bis 5 Gew.-%, bezogen auf das gesamte Haarfärbemittel, enthalten sind.

5. Haarfärbemittel nach den Ansprüchen 1 bis 4, dadurch gekennzeichnet, daß zusätzlich zu den Verbindungen der Formel I noch weitere direktziehende Haarfarbstoffe und/oder Oxidationsfarbstoffvorprodukte enthalten sind und daß diese Farbstoffe in einem kosmetischen Träger in Form einer Creme, einer Emulsion, eines Gels, eines Shampoos oder eines Schaumaerosols eingearbeitet sind.